# EUROPÄISCHE PATENTSCHRIFT

(11) **EP 1 089 761 B1**
(45) Veröffentlichungstag und Bekanntmachung des Hinweises auf die Patenterteilung: **25.09.2002**
(21) Anmeldenummer: 99941383.4
(22) Anmeldetag: 24.06.1999
(51) Int. Cl.: A61K 45/06, A61P 37/00

(54) **BISPHOSPHONSÄUREN UND DEREN DERIVATE ENTHALTENDE ARZNEIMITTEL ZUR PROPHYLAXE UND ZUR BEHANDLUNG VON AUTOIMMUNKRANKHEITEN SOWIE VON ALLERGIEN**
MEDICAMENTS CONTAINING BISPHOSPHONIC ACIDS AND DERIVATIVES THEREOF WHICH ARE PROVIDED FOR PREVENTING AND TREATING AUTOIMMUNE DISEASES AND ALLERGIES
MEDICAMENTS CONTENANT DES ACIDES BIPHOSPHONIQUES ET LEURS DERIVES POUR LA PROPHYLAXIE ET LE TRAITEMENT DE MALADIES AUTO-IMMUNES ET D'ALLERGIES

(30) Priorität: 26.06.1998 DE 19828450
(43) Veröffentlichungstag der Anmeldung: 11.04.2001
(73) Patentinhaber: Jomaa, Hassan, 35398 Giessen (DE)
(72) Erfinder: Jomaa, Hassan, 35398 Giessen (DE)
(74) Vertreter: Panten, Kirsten
(86) Internationale Anmeldenummer: DE9901844
(87) Internationale Veröffentlichungsnummer: WO00000182

(56) Entgegenhaltungen:
- WO-A-96/22790

## Beschreibung

### Technisches Gebiet

Die Erfindung betrifft die Verwendung bestimmter Bisphosphonsäuren sowie deren Derivaten zur Herstellung von Arzneimittel zur Prophylaxe und zur Behandlung von Autoimmunkrankheiten und von Allergien.

Es ist bekannt, daß Autoimmunkrankheiten, wie Multiple Sklerose, Rheumatoide Arthritis, Diabetes mellitus, Myasthenia gravis, Uveitis etc., sowie Allergien, insbesondere Nahrungsmittelallergien, Nickelallergie und Pollenallergien etc. auf eine Fehlreaktion des Immunsystems des Organismus zurückzuführen sind.

Es ist weiterhin bekannt, daß aufgrund dieser Fehlreaktionen das Immunsystem körpereigene Substanzen (die Autoantigene) als Fremdkörper auffaßt und dagegen eine Abwehrreaktion entwickelt, die zu einer Schädigung des körpereigenen Gewebes führt. Je nach dem beteiligten Organsystem sind etwa 40 Autoimmunkrankheiten bekannt geworden. Diese Abwehrreaktionen können sich sowohl gegen einzelne Zellbestandteile als auch gegen ganze Zellen bzw. Organe, richten.

Allergien beruhen bekanntlich auf einer Überempfindlichkeit gegenüber bestimmten Substanzen, den Allergenen, die zu einer Überreaktion des Immunsystems führen. Das heißt, daß die Betroffenen auf bestimmte Substanzen (die Allergene) mit spezifischen Symptomen zur Abwehr des Allergens reagieren.

### Stand der Technik

Versuche, die durch die Fehlreaktionen des Immunsystems erzeugten Autoimmunkrankheiten mit den unspezifisch wirkenden Immunsuppressiva zu behandeln, verliefen völlig unbefriedigend, da mit der Anwendung der Immunsuppressiva eine generelle Hemmung der entzündlichen Reaktionen bis hin zur Ausschaltung großer Teile des Immunsystems bewirkt wird, was das Auftreten vieler Nebenwirkungen, z.B. toxischer Schädigungen, erhöhter Anfälligkeit für Infektionskrankheiten und erhöhter Gefahr des Auftretens bösartiger Erkrankungen zur Folge hat.

Dem alternativen Versuch, die mit dem Einsatz der unspezifisch wirkenden Immunsuppressiva verbundenen Nebenwirkungen durch Anwendung einer selektiven Suppression (vgl. Ann. Neurol. 37 Suppl 1, 87-101) zu vermeiden, wobei gezielt und spezifisch an verschiedenen Stellen der Abwehrreaktion gegen die Allergene oder gegen die Autoantigene eingegriffen wird, war ebenfalls kein voller Erfolg beschieden.

Eine dieser Methoden beruht auf der oralen oder der inhalativen Verabreichung der krankheitsspezifischen Autoantigene oder Allergene. Es gelingt zwar auf diese Weise eine Reinduktion der Toleranz des Körpers gegen diese Autoantigene bzw. Allergene zu erzielen bzw. den Körper zu befähigen, die bislang angegriffenen Autoantigene oder Allergene zu tolerieren, die die gesteigerte Immunantwort auslösen. Insgesamt ist aber die Erfolgsrate dieser Desensibilisierung der Patienten begrenzt, weil die Desensibilisierung nicht ausreichend ist (Ann. N. Y. Acad. Sci. 778, 1-27; Ann. N. Y. Acad. Sci. 778, 243-250; Science 261, 1727-1730; Annu. Rev. Med. 48, 341-351).

Der Mechanismus der oralen Toleranz-Reinduktion durch diese Substanzen ist noch nicht gänzlich bekannt. Es ist aber anzunehmen, daß bei der oralen Applikation sowohl das Immunsystem als auch die bakterielle Besiedlung des Magen-Darm-Traktes, die T-Zellen (T-Lymphocyten), insbesondere die γδ-T-Zellen, eine Rolle spielen (The Journal of Immunology 158, 3610-3618; Res. Immunol. 147, 49-59; Immunology Letters 48, 97-102).

Es war jedenfalls völlig überraschend, daß die Reinduktion der mit der. oralen oder inhalativen Applikation der krankheitsspezifischen Autoantigene oder Allergene erzielte Toleranz stark gesteigert wurde, wenn die Autoantigene oder Allergene in Kombination mit Bisphosphonsäuren oder deren Derivaten angewandt wurden. Somit können diese Kombinationen mit Erfolg zur Prophylaxe und zur Behandlung der Autoimmunkrankheiten oder Allergien verwendet werden.

Die Verwendung von Bisphosphonsäuren sowie von einigen ihrer Derivate in Arzneimitteln ist bereits bekannt. Bislang ist die mikrobiostatische Wirksamkeit der Bisphosphonsäuren (DE 3611522), ihre Wirksamkeit bei der Behandlung von Störungen des Calcium- und Phosphatstoffwechsels (DE 2534390, DE 2534391, DE 3334211, DE 3434667, DE 2745083), die cytostatische Wirksamkeit (DE 3425812), ihre lipidsenkende Wirksamkeit (Arzneimittelforschung 46, 759-762) und deren Fähigkeit Immunzellen zu stimulieren (WO 97/38696 A1) bekannt. Weiter ist die Tatsache, daß Bisphosphonsäuren eine immunmodulatorische Wirkung aufweisen (WO 97/38696 A1), bekannt und genutzt worden.

Der Einsatz dieser Verbindungen ist aber mit vielen Nebenwirkungen verbunden, die von der Form der Applikation abhängig sind. Bei der intravenösen Infusion sind dies Fieber, grippeartige Symptome mit Schüttelfrost, Lymphopenie und Thrombozytopenie und bei der oralen Applikation sind es Schluckbeschwerden, Ösophagitis, Ösophagus-Erosionen, Ösophagus-Ulcus, Dysepsien, Durchfall etc.. Außerdem erfordert z.B. die orale Behandlung mit Bisphosphonaten relativ große Mengen an Wirkstoff und der Therapieerfolg ist trotzdem unbefriedigend (Drug-Saf. 14, 158-170).

Es war daher keineswegs naheliegend, diese Verbindungsgruppe in Kombination mit Autoantigenen oder Allergenen zur Reinduktion der Toleranz des Körpers gegen die Autoantigene bzw. Allergene zu benutzen.

### Darstellung der Erfindung

Die Erfindung betrifft somit einen neuen Weg, das bislang ungelöste Problem der Prophylaxe und der Behandlung von Autoimmunkrankheiten und von Allergien mit Hilfe von Arzneimitteln zu lösen, nämlich die bislang zur Behandlung von Autoimmunerkrankheiten und von Allergien benutzten Autoantigene bzw. Allergene in Kombination mit Bisphosphonaten bzw. deren Derivaten einzusetzen.

Die Erfindung betrifft die Verwendung von Bisphosphonsäuren sowie deren Derivaten zur Herstellung von Arzneimitteln zur Prophylaxe und zur Behandlung von Autoimmunkrankheiten oder Allergien, wobei als Bisphosphonsäuren und deren Derivate solche der allgemeinen Formel: 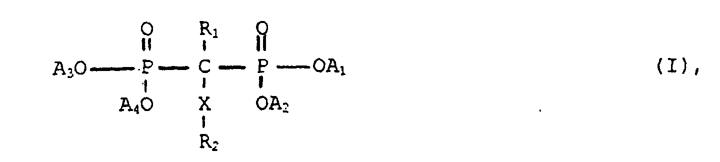 worin
- A₁,A₂,A₃,A₄,: die gleich oder verschieden sein können und Wasserstoff, substituiertes oder unsubstituiertes Alkyl, substituiertes oder unsubstituiertes Aryl, substituiertes oder unsubstituiertes Aralkyl, substituiertes oder unsubstituiertes Cycloalkyl oder einen substituierten oder unsubstituierten heterocyclischen Rest, ein Metall der 1., 2. oder 3. Hauptgruppe des periodischen Systems, wie Na, K, Ca, Mg, Al, sowie substituiertes oder unsubstituiertes Ammonium oder Ammoniumverbindungen, die sich von Ethylendiamin oder Aminosäuren ableiten, bedeuten,
- X,: das auch wegfallen kann oder Alkylen, Alkenylen oder Hydroxyalkylen sein kann,
- R₁, R₂,: die gleich oder verschieden sein können und H, OH, -NH₂, ein substituiertes oder unsubstituiertes Acyl, substituiertes oder unsubstituiertes Alkyl, substituiertes oder unsubstituiertes Aryl, substituiertes oder unsubstituiertes Cycloalkyl, substituiertes oder unsubstituiertes Aralkyl oder einen substituierten oder unsubstituierten heterocyclischen Rest oder -SR₃, Cl, und -NR₃R₄ bedeuten, worin
- R₃, R₄,: die gleich oder verschieden sein können und H, OH, substituiertes oder unsubstituiertes Acyl, substituiertes oder unsubstituiertes Alkyl, substituiertes oder unsubstituiertes Aryl, substituiertes oder unsubstituiertes Aralkyl, substituiertes oder unsubstituiertes Cycloalkyl oder einen substituierten oder unsubstituierten heterocyclischen Rest bedeuten,
und deren pharmazeutisch verträgliche Salze, Ester sowie Salze der Ester oder Verbindungen, die bei Applikation die zu verabreichenden Verbindungen als Stoffwechsel- oder Abbauprodukte bilden, in Kombination mit den für die Prophylaxe und für die Behandlung der jeweiligen Autoimmunkrankheit spezifischen Autoantigenen oder in Kombination mit den für die Prophylaxe und die Behandlung der jeweiligen Allergie spezifischen Allergenen verwendet werden,
wobei anstelle der jeweiligen Autoantigene oder Allergene auch deren Fragmente oder Derivate sowie die Analoga oder deren Fragmente der Autoantigene oder Allergene verwendet werden können, sofern sie jeweils dieselben immunologischen Eigenschaften wie die entsprechenden Gesamtmoleküle aufweisen, und
wobei die Bisphosphonsäuren oder ihre Derivate und
die Autoantigene oder Allergene, bzw. deren Fragmente, Derivate oder Analoga
gleichzeitig oder nacheinander appliziert werden können.

Dabei kann die Verabreichung der Substanzen sowohl synchron als auch zeitlich versetzt durch gleichzeitige oder getrennte Applikation der Wirkstoffe erfolgen.

Aus der Gruppe der Bisphosphonsäuren und deren Derivaten der allgemeinen Formel I wird für die Verwendung zur Prophylaxe und zur Behandlung von Autoimmunkrankheiten oder Allergien, den Bisphosphonsäuren und deren Derivaten der allgemeinen Formel: 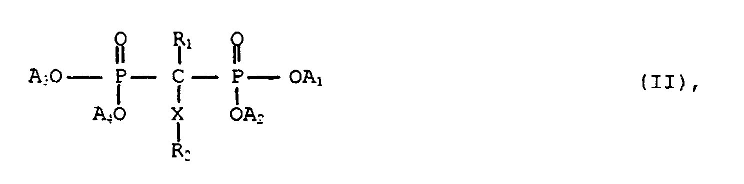 worin
- A₁, A₂, A₃, A₄,: die gleich oder verschieden sein können und Wasserstoff, einen Alkylrest mit 1 bis 12 Kohlenstoffatomen, der verzweigt oder unverzweigt sein kann, einen Arylrest, einen Aralkylrest, Cycloalkylrest oder einen heterocyclischen Rest, wobei diese Reste noch durch funktionelle Gruppen substituiert sein können, ein Metall der 1., 2. oder 3. Hauptgruppe des periodischen Systems, wie Na, K, Ca, Mg, Al, sowie substituiertes oder unsubstituiertes Ammonium oder Ammoniumverbindungen, die sich von Ethylendiamin oder Aminosäuren ableiten, bedeuten,
- R₁,: H, OH, NH₂,
- X,: das auch wegfallen kann, oder Alkylen, Alkenylen oder Hydroxyalkylen, mit jeweils 1 bis 12 Kohlenstoffatomen sein kann,
- R₂,: H, OH, NH₂, ein Alkylrest mit 1 bis 12 Kohlenstoffatomen, der verzweigt oder unverzweigt sein kann, einen Arylrest, Aralkylrest, Cycloalkylrest oder einen heterocyclischen Rest, wobei diese Reste noch durch funktionelle Gruppen substituiert sein können, oder -SR₃, Cl, und -NR₃R₄ bedeuten, worin
- R₃, R₄,: die gleich oder verschieden sein können und H, OH, einen Alkylrest mit 1 bis 12 Kohlenstoffatomen, der verzweigt oder unverzweigt sein kann, einen Arylrest, Aralkylrest, Cycloalkylrest oder einen heterocyclischer Rest, wobei diese Reste noch durch funktionelle Gruppen substituiert sein können bedeuten,
der Vorzug gegeben.

Als besonders wirksam haben sich Bisphosphonsäuren und deren Derivate der allgemeinen Formel: 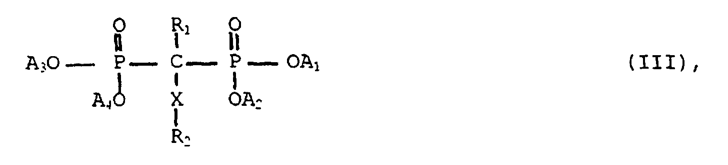 worin
- A₁, A₂, A₃, A₄,: die gleich oder verschieden sein können und Wasserstoff, einen Alkylrest mit 1 bis 12 Kohlenstoffatomen, der verzweigt oder unverzweigt sein kann, einen Arylrest, Aralkylrest, Cycloalkylrest oder einen heterocyclischen Rest, wobei diese Reste noch durch funktionelle Gruppen substituiert sein können, ein Metall der 1., 2. oder 3. Hauptgruppe des periodischen Systems, wie Na, K, Ca, Mg, Al sowie substituiertes oder unsubstituiertes Ammonium oder Ammoniumverbindungen, die sich von Ethylendiamin oder Aminosäuren ableiten, bedeuten,
- R₁,: H, OH,
- X,: das auch wegfallen oder (CH₂)₁₋₅, Amidino,
- R₂,: -NH₂ 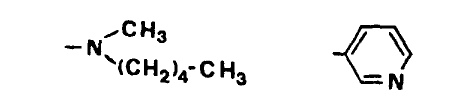 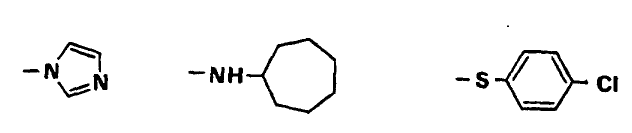
bedeuten kann, erwiesen.

Einige Beispiele hierfür sind:
Amino-hydroxy-methyliden-bisphosphonsäure (AMP),
2-Amino-1-hydroxyethyliden-1,1-bisphosphonsäure (AEP),
3-Amino-1-hydroxypropyliden-1,1-bisphosphonsäure (Pamidronsäure),
4-Amino-1-hydroxybutyliden-1,1-bisphosphonsäure (Alendronsäure),
6-Amino-1-hydroxyhexyliden-1,1-bisphosphonsäure (AHP), Amidinomethylen-bisphosphonsäure (AIMP),
3-Methylpentylamino-1-hydroxy-propyliden-1,1-bisphosphonsäure (Ibandronsäure),
2-(3-Pyridinyl)-1-hydroxyethyliden-bisphosphonsäure (Risedronsäure),
1-Hydroxy-2-(imidazol-1-yl)-ethyliden-1,1-bisphosphonsäure (Zoledronsäure),
Cycloheptyl-aminomethylendiphosphonsäure (Cimadronsäure),
4-Chlorphenyl-thiomethylen-1,1-bisphosphonsäure (Tiludronsäure) sowie deren Derivate erwiesen.

Die Prophylaxe und die Behandlung von Autoimmunkrankheiten und Allergien erfolgt durch kombinierte Anwendung einer Bisphosphonsäure oder ihren Derivaten und einem, die jeweilige Autoimmunkrankheit auslösenden Autoantigen wie beispielsweise bei
- Multipler Sklerose: mit Myelin Basischen Protein (MBP), weiteren Extrakten aus dem Gewebe des Nervensystems,
- Rheumatoide Arthritis: mit Kollagen des Typs I, II oder III,
- Hashimoto-Thyreoiditis: mit Thyreoglobulin,
- Myasthenia gravis: mit Acetylcholinrezeptorprotein,
- Lupus erythematodes: mit DNS,
- Diabetes mellitus: mit Inselzell-Extrakten, Humaninsulin,
- Primär-biliäre Zirrhose: mit Leberextrakten
- Aktive chronische Hepatitis: mit Leberzellextrakten,
- Adrenalitis/Addison-Krankheit: mit Nebennierenrindenextrakten,
- Polymyositis: mit Hautextrakten, Muskelextrakten
- Dermatomyositis: mit Muskel- und/oder Hautextrakten,
- Autoimmunhämolytische Anämie: mit Extrakten aus Zellen der hämatopoetischen Linie,
- Herzmuskel-, Herzhautentzündung: mit Herzextrakten,
- Sklerodermie: mit Hautextrakten, Hautzellextrakten,
- Uveitis (Phakouveitis, sympathische Ophthalmie): mit Augenlinsenproteinen, S-Antigenen, S-Antigengemischen,
- Pemphigus vulgaris: mit Hautextrakten,
- Pemphigoid: mit Hautextrakten,
- Perniziöse Anämie: mit Magenzellextrakten, Parietalzellextrakten, Intrinsischen Faktor,
- Autoimmune atrophische Gastritis: mit Magenzellextrakten,
- Crohn-Krankheit: mit Darmextrakten,
- Colitis ulcerosa: mit Darmextrakten
- oder bei Allergien: mit dem allergiespezifischen Allergen.

Als kombinierte Anwendung werden auch die Fälle verstanden, bei denen Autoantigene oder Allergene bereits vorhanden sind. Dazu gehört z.B. Morbus Crohn, bei dem das Autoantigen krankheitsbedingt bereits im Darm vorhanden ist. In diesem Fall müssen bei oraler oder rektaler Applikation nur die Bisphosphonsäuren oder ihre Derivate verabreicht werden. Ebenso entfällt die Verabreichung des Allergens, wenn der Betroffene sich während der Therapie in einer Umgebung befindet, in der das allergiespezifische Allergen bereits vorhanden ist (z.B. zur Pollenflugzeit die Pollen).

Die kombinierte Anwendung kann nicht nur oral, z.B. mittels Tabletten etc., erfolgen, sondern kann beispielsweise auch rektal, inhalativ, durch Auftragen auf Haut oder Schleimhäute verabreicht werden. Bevorzugte Anwendungsformen sind die orale und die inhalative Applikation sowie das Auftragen auf Haut oder Schleimhäute.

Von diesen Anwendungformen hat sich die Inhalation als eine besonders schonende Anwendungsform erwiesen, da bereits mit sehr geringen Mengen an Autoantigen oder Allergen und Bisphosphonsäure oder ihren Derivaten eine hohe Wirksamkeit erzielt wird und damit eventuelle Nebenwirkungen der Wirkstoffe gering gehalten werden.

Von den Bisphosphonsäuren und ihren Derivaten werden bevorzugt solche angewandt, die schlecht resorbiert werden, wozu z.B. die Aminobisphosphonsäuren und ihre Derivate gehören.

Bevorzugte pharmazeutische Zusammensetzungen sind Tabletten, Dragees, Kapseln, Pillen, Granulate, Suppositorien, Lösungen, Suspensionen und Emulsionen, Pasten, Salben, Gele, Cremes, Lotionen, Puder und Sprays. Tabletten, Dragees, Kapseln, Pillen und Granulate können den oder die Wirkstoffe neben den üblichen Trägerstoffen enthalten, wie (a) Füllund Streckmittel, z. B. Stärke, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z. B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quaternäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden, Überzügen und Hüllen versehen und so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. Cij-Alkohol mit C₁₆-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilicat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe, wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem oder den Wirkstoffen die üblichen Trägerstoffe, wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z.B. Saccharin, enthalten.

Bisphosphonsäuren oder ihre Derivate der Formel (I) eignen sich zur gleichzeitigen, getrennten oder zeitlich abgestuften Anwendung mit den Autoantigenen oder Allergenen, daher sollten diese Verbindungen in den oben aufgeführten pharmazeutischen Zubereitungen, vorzugsweise in einer Konzentration von etwa 0,1 bis 99,5 Gew.%, der Gesamtmischung vorhanden sein. Die Konzentration der Autoantigene oder Allergene soll auch hier 0.1 bis 99,5 Gew.% betragen.

Die oben aufgeführten pharmazeutischen Zubereitungen können außer den Verbindungen der Formel (I) und dem Autoantigen oder Allergen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die genannten Zubereitungen können bei Mensch oder Tier entweder oral, rektal, intravaginal, lokal (Puder, Salbe, Tropfen) und in Hohlräumen und Körperhöhlen angewendet werden. Als geeignete Zubereitungen kommen Lösungen und Suspensionen für die orale Therapie, Gele, Aufgußformulierungen, Emulsionen, Salben oder Tropfen in Frage. Zur lokalen Therapie können ophthalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneter Formulierung erfolgen. Ferner können Gele, Pulver, Puder, Tabletten, Retard-Tabletten, Premixe, Konzentrate, Granulate, Pellets, Boli, Kapseln, Aerosole, Sprays, Inhalate bei Mensch und Tier angewendet werden. Ferner können die erfindungsgemäßen Verbindungen in andere Trägermaterialien, wie zum Beispiel Kunststoffe (Kunststoffketten zur lokalen Therapie), Kollagen oder Knochenzement eingearbeitet werden.

Die notwendigen Mengen der einzelnen Derivate zur Erzielung des gewünschten Effektes unterscheiden sich sehr stark. Im allgemeinen hat es sich sowohl in der Human- als auch in der Veterinärmedizin als vorteilhaft erwiesen, den oder die Wirkstoffe der Formel (I) in Gesamtmengen von etwa 0,5 bis etwa 2000 mg je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 0,5 bis etwa 2000 mg. Es kann jedoch erforderlich sein, von den genannten Dosierungen abzuweichen, und zwar in Abhängigkeit von der Art und dem Körpergewicht des zu behandelnden Patienten, der Art und Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch den Fachmann aufgrund seines Fachwissens erfolgen.

Bei der Behandlung von Tieren können die erfindungsgemäß zu verwendenden Verbindungen in den üblichen Konzentrationen und Zubereitungen zusammen mit dem Futter bzw. mit Futterzubereitungen oder mit dem Trinkwasser gegeben werden.

### Beispiele

Zur Herstellung von Tabletten in an sich bekannter Weise wird eine Mischung von

| 1. 3-Amino-1-hydroxypropyliden-1,1-bisphosphonat, | |
|---|---|
| Dinatriumsalz, | 600 mg |
| Bovines Kollagen Typ II | 10 mg |
| Mannit | 400 mg |
| Stärke | 50 mg |
| Magnesiumstearat | 10 mg |

| 2. 4-Amino-1-hydroxybutyliden-1,1-bisphosphonat | |
|---|---|
| (Mononatriumsalz) 3H₂O | 26 mg |
| Bovines Kollagen Typ II | 10 mg |
| Mannit | 400 mg |
| Stärke | 50 mg |
| Magnesiumstearat | 10 mg |

| 3. 3-Methylpentylamino-1-hydroxypropyliden-1,1-bisphosphonat | |
|---|---|
| Mononatriumsalz 1H₂O | 1,125 mg |
| Bovines Kollagen Typ II | 10 mg |
| Mannit | 400 mg |
| Stärke | 50 mg |
| Magnesiumstearat | 10 mg |

| 4. 3-Amino-1-hydroxypropyliden-1,1-bisphosphonat, | |
|---|---|
| Dinatriumsalz, | 600 mg |
| Myelin Basisches Protein (MBP) | 8 mg |
| Mannit | 400 mg |
| Stärke | 50 mg |
| Magnesiumstearat | 10 mg |

| 5. 4-Amino-1-hydroxybutyliden-1,1-bisphosphonat | |
|---|---|
| (Mononatriumsalz) 3H₂O | 26 mg |
| Myelin Basisches Protein (MBP) | 8 mg |
| Mannit | 400 mg |
| Stärke | 50 mg |
| Magnesiumstearat | 10 mg |

| 6. 3-Methylpentylamino-1-hydroxypropyliden-1,1-bisphosphonat | |
|---|---|
| Mononatriumsalz 1H₂O | 1,125 mg |
| Myelin Basisches Protein (MBP) | 8 mg |
| Mannit | 400 mg |
| Stärke | 50 mg |
| Magnesiumstearat | 10 mg |

verwendet.

Zur Herstellung von Kapseln in an sich bekannter Weise werden

| 7. 3-Amino-1-hydroxypropyliden-1,1-bisphosphonat, | |
|---|---|
| Dinatriumsalz, | 600 mg |
| Myelin Basisches Protein (MBP) | 8 mg |
| Proteolipid Protein | 15 mg |
| Magnesiumstearat | 15 mg |

| 8. 4-Amino-1-hydroxybutyliden-1,1-bisphosphonat | |
|---|---|
| (Mononatriumsalz) 3H₂O | 26 mg |
| Myelin Basisches Protein (MBP) | 8 mg |
| Proteolipid Protein | 15 mg |
| Magnesiumstearat | 15 mg |

| 9. 3-Methylpentylamino-1-hydroxypropyliden-1,1-bisphosphonat | |
|---|---|
| Mononatriumsalz 1H₂O | 1,125 mg |
| Myelin Basisches Protein (MBP) | 8 mg |
| Proteolipid Protein | 15 mg |
| Magnesiumstearat | 15 mg |

| 10. 3-Amino-1-hydroxypropyliden-1,1-bisphosphonat, | |
|---|---|
| Dinatriumsalz, | 600 mg |
| Bovines Kollagen Typ II | 10 mg |
| Magnesiumstearat | 15 mg |

| 11. 4-Amino-1-hydroxybutyliden-1,1-bisphosphonat | |
|---|---|
| (Mononatriumsalz) 3H₂O | 26 mg |
| Bovines Kollagen Typ II | 10 mg |
| Magnesiumstearat | 15 mg |

| 12. 3-Methylpentylamino-1-hydroxypropyliden-1,1-bisphosphonat | |
|---|---|
| Mononatriumsalz 1H₂O | 1,125 mg |
| Bovines Kollagen Typ II | 10 mg |
| Magnesiumstearat | 15 mg |

verwendet, wobei
die vorstehenden Bestandteile miteinander vermischt und dann in eine harte Gelatinekapsel in herkömmlicher Weise eingebracht werden.

Zur Herstellung einer Zubereitung zur Inhalation für eine Dosis von 2 ml werden:

| 13. 4-Amino-1-hydroxybutyliden-1,1-bisphosphonat | |
|---|---|
| (Mononatriumsalz) 3H₂O | 1,3 mg |
| Myelin Basisches Protein | 15 mg |
| β-Cyclodextrinhydrat | 7 mg |
| pH 7,2 Phosphatpuffer zur Injektion geeignetes Wasser; | 0,2 ml |

| 14. 3-Amino-1-hydroxypropyliden-1,1-bisphosphonat, | |
|---|---|
| Dinatriumsalz, | 30 mg |
| Myelin Basisches Protein | 15 mg |
| β-Cyclodextrinhydrat | 7 mg |
| pH 7,2 Phosphatpuffer zur Injektion geeignetes Wasser; | 0,2 ml |

| 15. 3-Methylpentylamino-1-hydroxypropyliden-1,1-bisphosphonat | |
|---|---|
| Mononatriumsalz 1H₂O | 0,25 mg |
| Myelin Basisches Protein | 15 mg |
| β-Cyclodextrinhydrat | 7 mg |
| pH 7,2 Phosphatpuffer zur Injektion geeignetes Wasser; | 0,2 ml |

| 16. 4-Amino-1-hydroxybutyliden-1,1-bisphosphonat | |
|---|---|
| (Mononatriumsalz) 3H₂O | 1,3 mg |
| Bovines Kollagen Typ II | 10 mg |
| β-Cyclodextrinhydrat | 7 mg |
| pH 7,2 Phosphatpuffer zur Injektion geeignetes Wasser; | 0,2 ml |

| 17. 3-Amino-1-hydroxypropyliden-1,1-bisphosphonat, | |
|---|---|
| Dinatriumsalz, | 30 mg |
| Bovines Kollagen Typ II | 10 mg |
| β-Cyclodextrinhydrat | 7 mg |
| pH 7,2 Phosphatpuffer zur Injektion geeignetes Wasser; | 0,2 ml |

| 18. 3-Methylpentylamino-1-hydroxypropyliden-1,1-bisphosphonat | |
|---|---|
| Mononatriumsalz 1H₂O | 0,25 mg |
| Bovines Kollagen Typ II | 10 mg |
| β-Cyclodextrinhydrat | 7 mg |
| pH 7,2 Phosphatpuffer zur Injektion geeignetes Wasser | 0,2 ml |

verwendet.

Das Bisphosphonat (z.B. Alendronat) und das Autoantigen (z.B. MBP) werden in einer Phosphatpufferlösung gelöst und darin das β-cyclodextrin-hydrat aufgelöst. Die Lösung wird mit zur Injektion geeignetem Wasser auf das gewünschte Volumen aufgefüllt, durch Filtrieren sterilisiert und aseptisch in Behälter, die für Inhalation durch Zerstäubung geeignet sind, gefüllt.

## Patentansprüche

1. Verwendung von Bisphosphonsäuren sowie deren Derivaten zur Herstellung von Arzneimitteln zur Prophylaxe und/oder zur Behandlung von Autoimmunkrankheiten und/oder von Allergien, **dadurch gekennzeichnet, daß** als Bisphosphonsäuren und deren Derivate verwendet werden:
- Bisphosphonsäuren und deren Derivate der allgemeinen Formel 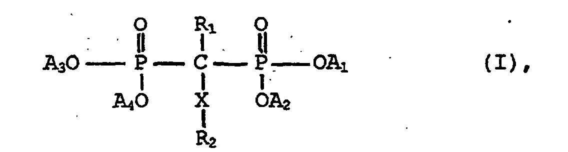 worin bedeuten können:
A₁,A₂,A₃,A₄, die gleich oder verschieden sein können: Wasserstoff, substituiertes oder unsubstituiertes Alkyl, substituiertes oder unsubstituiertes Aryl, substituiertes oder unsubstituiertes Aralkyl, substituiertes oder unsubstituiertes Cycloalkyl, einen substituierten oder unsubstituierten heterocyclischen Rest, ein Metall der 1., 2. oder 3. Hauptgruppe des Periodensystems, wie Na, K, Ca, Mg, Al, sowie substituiertes oder unsubstituiertes Ammonium oder Ammoniumverbindungen, die sich von Ethylendiamin oder Aminosäuren ableiten;
X, das auch wegfallen kann: Alkylen, Alkenylen oder Hydroxyalkylen;
R₁,R₂, die gleich oder verschieden sein können und bedeuten können: H, OH, NH₂, substituiertes oder unsubstituiertes Acyl, substituiertes oder unsubstituiertes Alkyl, substituiertes oder unsubstituiertes Aryl, substituiertes oder unsubstituiertes Cycloalkyl, substituiertes oder unsubstituiertes Aralkyl oder einen substituierten oder unsubstituierten heterocyclischen Rest und ferner SR₃, Cl, und NR₃R₄, worin
R₃,R₄ gleich oder verschieden sein können und bedeuten können: H, OH, substituiertes oder unsubstituiertes Acyl, substituiertes oder unsubstituiertes Alkyl, substituiertes oder unsubstituiertes Aryl, substituiertes oder unsubstituiertes Aralkyl, substituiertes oder unsubstituiertes Cycloalkyl oder einen substituierten oder unsubstituierten heterocyclischen Rest;
- und/oder pharmazeutisch verträgliche Salze und Ester der Bisphosphonsäuren sowie Salze der Ester
- und/oder Verbindungen, die nach ihrer Verabreichung im Körper zu Stoffwechsel- oder Abbauprodukten führen, welche den Bisphosphonsäuren bzw. deren vorgenannten Derivaten entsprechen,
wobei die Verwendung der Bisphosphonsäuren und/oder deren Derivate und/oder der zu den geeigneten Stoffwechsel- oder Abbauprodukten führenden Verbindungen
in Kombination mit den für die Prophylaxe und. für die Behandlung der jeweiligen Autoimmunkrankheit spezifischen Autoantigenen oder
in Kombination mit den für die Prophylaxe und die Behandlung der jeweiligen Allergie spezifischen Allergenen
erfolgt, wobei anstelle der jeweiligen Autoantigene bzw. Allergene auch Fragmente, Derivate oder Analoga der Autoantigene bzw. Allergene und ferner Fragmente der Analoga verwendet werden können, sofern die genannten Substanzen jeweils dieselben immunologischen Eigenschaften wie die entsprechenden Gesamtmoleküle der Autoantigene bzw. Allergene aufweisen.

2. Verwendung von Bisphosphonsäuren sowie deren Derivaten nach Anspruch 1, **dadurch gekennzeichnet, daß** in der allgemeinen Formel (I) bedeuten können:
A₁,A₂,A₃,A₄, die gleich oder verschieden sein können: Wasserstoff, einen verzweigten oder unverzweigten Alkylrest mit 1 bis 12 Kohlenstoffatomen, ein Arylrest, Aralkylrest, Cycloalkylrest oder einen heterocyclischen Rest, wobei diese Reste durch funktionelle Gruppen substituiert sein können, ein Metall der 1., 2. oder 3. Hauptgruppe des Periodensystems, wie Na, K, Ca, Mg, Al, sowie substituiertes oder unsubstituiertes Ammonium oder Ammoniumverbindungen, die sich von Ethylendiamin oder Aminosäuren ableiten;
R₁: H, OH, NH₂;
X, das auch wegfallen kann: Alkylen, Alkenylen oder Hydroxyalkylen, mit jeweils 1 bis 12 Kohlenstoffatomen;
R₂: H, OH, NH₂, einen verzweigten oder unverzweigten Alkylrest mit 1 bis 12 Kohlenstoffatomen, einen Arylrest, Aralkylrest, Cycloalkylrest oder einen heterocyclischen Rest, wobei diese Reste durch funktionelle Gruppen substituiert sein können, und ferner SR_{3,} Cl, und NR₃R₄, worin
R₃,R₄, gleich oder verschieden sein können und bedeuten können: H, OH, einen verzweigten oder unverzweigten Alkylrest mit 1 bis 12 Kohlenstoffatomen, einen Arylrest, Aralkylrest, Cycloalkylrest oder einen heterocyclischen Rest, wobei diese Reste durch funktionelle Gruppen substituiert sein können.

3. Verwendung von Bisphosphonsäuren sowie deren Derivaten zur Herstellung von Arzneimitteln zur Prophylaxe und/oder zur Behandlung von Autoimmunkrankheiten und/oder Allergien, **dadurch gekennzeichnet, daß** als Bisphosphonsäuren und deren Derivate verwendet werden:
- Bisphosphponsäuren und deren Derivate der allgemeinen Formel 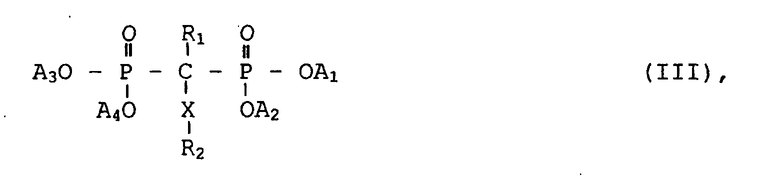 worin bedeuten können:
A₁, A₂, A₃, A₄, die gleich oder verschieden sein können:
Wasserstoff, einen verzweigten oder unverzweigten Alkylrest mit 1-12 Kohlenstoffatomen, einen Arylrest, Aralkylrest, Cycloalkylrest oder einen heterocyclischen Rest, wobei diese Reste durch funktionelle Gruppen substituiert sein können, ein Metall der 1., 2. oder 3. Hauptgruppe des Periodensystems, wie Na, K, Ca, Mg, Al, sowie substituiertes oder unsubstituiertes Ammonium oder Ammoniumverbindungen, die sich von Ethylendiamin oder Aminosäuren ableiten,
R₁: H, OH,
X, das auch wegfallen kann: (CH₂)₁₋₅, Amidino,
R₂: -NH₂ 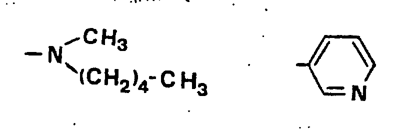 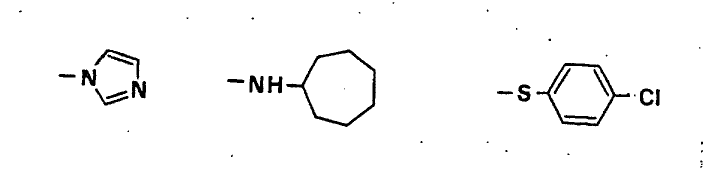
wobei die Verwendung der Bisphosphonsäuren und/oder deren Derivate und/oder der zu den geeigneten Stoffwechsel- oder Abbauprodukten führenden Verbindungen
in Kombination mit den für die Prophylaxe und die Behandlung der jeweiligen Autoimmunkrankheit spezifischen Autoantigenen oder
in Kombination mit den für die Prophylaxe und die Behandlung der jeweiligen Allergie spezifischen Allergenen erfolgt, wobei anstelle der jeweiligen Autoantigene bzw. Allergene auch Fragmente, Derivate oder Analoga der Autoantigene bzw. Allergene und ferner Fragmente der Analoga verwendet werden können, sofern die genannten Substanzen jeweils dieselben immunologischen Eigenschaften wie die entsprechenden Gesamtmoleküle der Autoantigene bzw. Allergenen aufweisen.

4. verwendung von Bisphosphonsäuren und deren Derivaten in Kombination mit Autoantigenen bzw. Allergenen zur Herstellung von Arzneimitteln für die Prophylaxe und/oder die Behandlung von Autoimmunkrankheiten und/oder von Allergien nach einem der Ansprüche 1 bis 3, **dadurch gekennzeichnet, daß** verwendet werden zur Behandlung von
Multipler Sklerose: Myelin Basisches Protein (MBP), weitere Extrakte aus dem Gewebe des Nervensystems;
Rheumatoider Arthritis: Kollagen der Typen I, II, III;
Hashimoto-Thyreoiditis: Thyreoglobulin;
Myasthenia gravis: Acetylcholinrezeptorprotein;
Lupus erythematodes: DNS;
Diabetes mellitus: Inselzell-Extrakte, Humaninsulin;
Primär-biliärer Zirrhose: Leberextrakte;
Aktiver chronischer Hepatitis: Leberzellextrakte;
Adrenalitis/Addison-Krankheit: Nebennierenrindenextrakte;
Polymyositis: Hautextrakte,Muskelextrakte;
Dermatomyositis: Muskel- und/oder Hautextrakte;
Autoimmunhämolytischer Anämie: Extrakte aus Zellen der häma topoetischen Linie;
Herzmuskel-, Herzhautentzündung: Herzextrakte;
Sklerodermie: Hautextrakte, Hautzellextrakte;
Uveitis (Phakouveitis, Augenlinsenproteine;
sympathische Ophthalmie): S-Antigene,
S-Antigengemische;
Pemphigus vulgaris: Hautextrakte;
Pemphigoid: Hautextrakte;
Perniziöser Anämie: Magenzellextrakte,
Parietalzellextrakte,
Intrinsischer Faktor;
Autoimmuner atrophischer Gastritis: Magenzellextrakte;
Crohn-Krankheit: Darmextrakte;
Colitis-ulcerosa-Allergien: Darmextrakte,
allergiespezifische Allergene.

5. Verwendung von Bisphosphonsäuren und deren Derivaten zur Herstellung von Arzneimitteln für die Prophylaxe und/oder die Behandlung von Autoimmunkrankheiten und/oder von Allergien nach einem der Ansprüche 1 bis 3, **dadurch gekennzeichnet, daß** die Zubereitungen der Kombinationen in fester Form, als Salbe, als Lösung oder als Spray angewendet werden.

## Claims

1. Use of bisphosphonic acids and the derivatives thereof for the production of pharmaceutical preparations for prevention and/or treatment of autoimmune diseases and/or allergies, **characterised in that** the bisphosphonic acids and their derivatives:
- bisphosphonic acids and their derivatives of general formula: 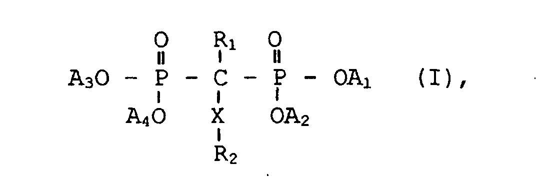 are used in which
A₁, A₂, A₃, A₄, which may be identical or different may mean hydrogen, substituted or unsubstituted alkyl, substituted or unsubstituted aryl, substituted or unsubstituted aralkyl, substituted or unsubstituted cycloalkyl or a substituted or unsubstituted heterocyclic residue, a metal of main group 1, 2 or 3 of the periodic system, such as Na, K, Ca, Mg, Al, as well as substituted or unsubstituted ammonium or ammonium compounds derived from ethylenediamine or amino acids,
X, which may also be absent or may be alkylene, alkenylene or hydroxyalkylene,
R₁, R₂, which may be identical or different and mean H, OH, -NH₂, substituted or unsubstituted acyl, substituted or unsubstituted alkyl, substituted or unsubstituted aryl, substituted or unsubstituted cycloalkyl, substituted or unsubstituted aralkyl or a substituted or unsubstituted heterocyclic residue or -SR₃, Cl and -NR₃R₄, in which
R₃,R₄, which may be identical or different and mean H, OH, substituted or unsubstituted acyl, substituted or unsubstituted alkyl, substituted or unsubstituted aryl, substituted or unsubstituted aralkyl, substituted or unsubstituted cycloalkyl or a substituted or unsubstituted heterocyclic residue,
- and/or pharmaceutically compatible salts, esters of the bisphosphonic acids as well as salts of esters
- and/or compounds which, upon administration, form the compounds to be administered as metabolites or catabolites, which are identical to the above cited bisphosphonic acids and derivatives thereof,
wherein the use of the bisphosphonic acids and/or their derivatives and/or the compounds forming the suitable metabolites or catabolites is performed
in combination with the specific autoantigens for the prevention and treatment of the particular autoimmune disorder, or
in combination with the specific allergens for the prevention and treatment of the particular allergy, wherein, instead of the particular autoantigens or allergens, it is also possible to use fragments or derivatives or analogues of the autoantigens or allergens and the analogues and fragments of the autoantigens or allergens, providing that the listed substances exhibit the same immunological characteristics as the corresponding whole molecules of the autoantigens or allergens.

2. Use of bisphosphonic acids and the derivatives thereof according to claim 1, **characterised in that**, in general formula (I):
A₁, A₂, A₃, A₄, which may be identical or different may mean hydrogen, an alkyl residue having 1 to 12 carbon atoms, which may be branched or unbranched, an aryl residue, aralkyl residue, cycloalkyl residue or a heterocyclic residue, wherein these residues may additionally be substituted by functional groups, a metal of main group 1, 2 or 3 of the periodic system, such as Na, K, Ca, Mg, Al, as well as substituted or unsubstituted ammonium or ammonium compounds derived from ethylenediamine or amino acids,
R₁, means H, OH, NH₂,
X, which may also be absent or may be alkylene, alkenylene or hydroxyalkylene, in each case having 1 to 12 carbon atoms,
R₂, means H, OH, -NH₂, an alkyl residue having 1 to 12 carbon atoms, which may be branched or unbranched, an aryl residue, aralkyl residue, cycloalkyl residue or a heterocyclic residue, wherein these residues may additionally be substituted by functional groups, or -SR₃, Cl and
-NR₃R₄, in which
R₃, R₄, which may be identical or different and mean H, OH, an alkyl residue having 1 to 12 carbon atoms, which may be branched or unbranched, an aryl residue, aralkyl residue, cycloalkyl residue or a heterocyclic residue, wherein these residues may additionally be substituted by functional groups.

3. Use of bisphosphonic acids and the derivatives thereof in combination with autoantigens or allergens for the production of pharmaceutical preparations for the prevention and treatment of autoimmune diseases and/or allergies according to one of claims 1 to 3, **characterised in that** as bisphosphonic acids and the derivatives thereof, bisphosphonic acids and their derivatives of the general formula: 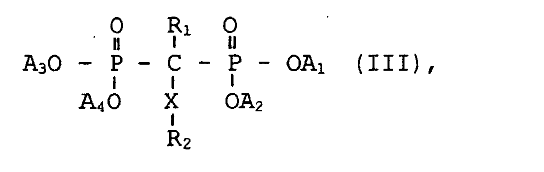 are used in which
A₁, A₂, A₃, A₄, which may be identical or different and mean hydrogen, an alkyl residue having 1 to 12 carbon atoms, which may be branched or unbranched, an aryl residue, aralkyl residue, cycloalkyl residue or a heterocyclic residue, wherein these residues may additionally be substituted by functional groups, a metal of main group 1, 2 or 3 of the periodic system, such as Na, K, Ca, Mg, Al, as well as substituted or unsubstituted ammonium or ammonium compounds derived from ethylenediamine or amino acids,
R₁, means OH,
X, which may also be absent or may mean (CH₂)₁₋₅, amidino,
R₂, may mean
-NH₂ 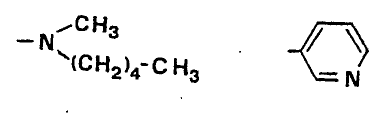 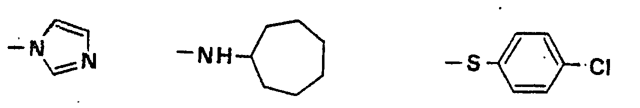
wherein the use of the bisphosphonic acids and/or their derivatives and/or the compounds forming the suitable metabolites or catabolites is performed
in combination with the specific autoantigens for the prevention and treatment of the particular autoimmune disorder, or
in combination with the specific allergens for the prevention and treatment of the particular allergy, wherein, instead of the particular autoantigens or allergens, it is also possible to use fragments or derivatives or analogues of the autoantigens or allergens and the analogues and fragments of the autoantigens or allergens, providing that the listed substances exhibit the same immunological characteristics as the corresponding whole molecules of the autoantigens or allergens.

4. Use of bisphosphonic acids and the derivatives thereof in combination with autoantigens or allergens for the production of pharmaceutical preparations for the prevention and treatment of autoimmune diseases and/or allergies according to one of claims 1 to 3, **characterised in that** the following are used for the treatment of
multiple sclerosis myelin basic protein (MBP), further extracts from nervous system tissue,
rheumatoid arthritis type I, II or III collagen,
Hashimoto thyroiditis thyroglobulin,
myasthenia gravis acetylcholine receptor protein,
lupus erythematosus DNA,
diabetes mellitus islet cell extracts, human insulin,
primary biliary cirrhosis liver extracts,
active chronic hepatitis liver cell extracts,
adrenalitis/Addison's disease adrenal cortex extracts,
polymyositis skin extracts, muscle extracts,
dermatomyositis muscle and/or skin extracts,
autoimmune haemolytic anaemia haemopoetic cell line extracts,
myocarditis, heart extracts,
myopericarditis scleroderma skin extracts, skin cell extracts,
uveitis (phacouveitis, eye lens proteins,
sympathetic ophthalmia) S-antigens, S-antigen mixtures,
pemphigus vulgaris skin extracts,
pemphigoid skin extracts,
pernicious anaemia gastric cell extracts,
parietal cell extracts,
intrinsic factor,
autoimmune atrophic gastritis gastric cell extracts,
Crohn's disease intestinal extracts,
colitis ulcerosa intestinal extracts
allergies allergy-specific
allergens.

5. Use of bisphosphonic acids and the derivatives thereof for the production of pharmaceutical preparations for the prevention and/or treatment of autoimmune conditions and/or allergies according to one of claims 1 to 3, **characterised in that** the combination preparations are used in solid form, as ointments, as solutions or as sprays.

## Revendications

1. Utilisation d'acides bisphosphoniques et de leurs dérivés pour la fabrication de médicaments pour la prophylaxie et/ou le traitement de maladies autoimmunes et/ou d'allergies, dans laquelle sont utilisés comme acides bisphosphoniques et leurs dérivés :
- des acides bisphosphoniques et leurs dérivés de formule générale 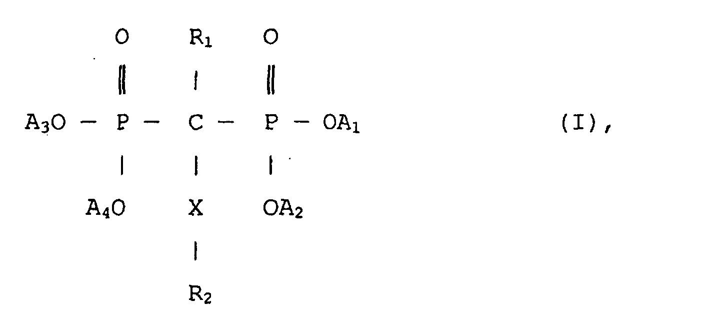 dans laquelle peuvent signifier :
A₁, A₂, A₃, A₄, qui peuvent être identiques ou différents : un hydrogène, alkyle substitué ou non substitué, aryle substitué ou non substitué, aralkyle substitué ou non substitué, cycloalkyle substitué ou non substitué, un reste hétérocyclique substitué ou non substitué, un métal du 1^{er}, 2^{ième} ou 3^{ième} groupe principal du système périodique, tels que Na, K, Ca, Mg, Al, de même qu'ammonium ou des composés d'ammonium substitué ou non substitué, qui sont dérivés de l'éthylènediamine ou d'acides aminés ;
X, qui peut également être supprimé : alkyles, alcényles ou hydroxyalkyles ;
R₁, R₂, qui peuvent être identiques ou différents et qui peuvent signifier : H, OH, NH₂, acyle substitué ou non substitué, alkyle substitué ou non substitué, aryle substitué ou non substitué, cycloalkyle substitué ou non substitué, aralkyle substitué ou non substitué ou un reste hétérocyclique substitué ou non substitué et en outre SR₃, Cl, et NR₃R₄, où
R₃, R₄ peuvent être identiques ou différents et peuvent signifier : H, OH, acyle substitué ou non substitué, alkyle substitué ou non substitué, aryle substitué ou non substitué, aralkyle substitué ou non substitué, cycloalkyle substitué ou non substitué ou un reste hétérocyclique substitué ou non substitué ;
- et/ou des sels pharmaceutiquement acceptables et des esters des acides bisphosphoniques de même que les sels des esters
- et/ou des composés, qui après leur administration dans le corps aboutissent à des produits métaboliques ou de dégradation, lesquels correspondent aux acides bisphosphoniques ou à leurs dérivés cités précédemment,
où l'utilisation des acides bisphosphoniques et/ou de leurs dérivés et/ou des composés aboutissant aux produits métaboliques ou de dégradation adéquats a lieu
en combinaison avec les auto-antigènes spécifiques pour la prophylaxie et le traitement de la maladie auto-immune correspondante ou
en combinaison avec les allergènes spécifiques pour la prophylaxie et le traitement de l'allergie correspondante
dans laquelle des fragments, dérivés ou analogues des auto-antigènes ou des allergènes ou encore des fragments d'analogues peuvent aussi être utilisés à la place des auto-antigènes ou des allergènes respectifs, dès lors que lesdites substances présentent respectivement les mêmes propriétés immunologiques que les molécules correspondantes des auto-antigènes ou allergènes prises dans leur ensemble.

2. Utilisation d'acides bisphosphoniques et de leurs dérivés selon la revendication 1, **caractérisée en ce que** peuvent signifier dans la formule générale (I) :
A₁, A₂, A₃, A₄, qui peuvent être identiques ou différents : hydrogène, un reste alkyle ramifié ou non ramifié avec de 1 à 12 atomes de carbone, un reste aryle, un reste aralkyle, un reste cycloalkyle ou un reste hétérocyclique, ces restes pouvant être substitués par des groupes fonctionnels, un métal du 1^{er}, 2^{ième} ou 3^{ième} groupe principal du système périodique, tels que Na, K, Ca, Mg , Al, de même qu'ammonium ou des composés d'ammonium substitué ou non substitué qui sont dérivés de l'éthylènediamine ou d'acides aminés ;
R₁ : H, OH, NH₂ ;
X, qui peut également être supprimé : alkyles, alcényles ou hydroxyalkyles, avec chacun de 1 à 12 atomes de carbone ;
R₂ : H, OH, NH₂, un reste alkyle ramifié ou non ramifié avec de 1 à 12 atomes de carbone, un reste aryle, un reste aralkyle, un reste cycloalkyle ou un reste hétérocyclique, ces restes pouvant être substitués par des groupes fonctionnels ou en outre SR₃, Cl, et NR₃R₄, où
R₃, R₄, peuvent être identiques ou différents et signifier : H, OH, un reste alkyle ramifié ou non ramifié avec de 1 à 12 atomes de carbone, un reste aryle, un reste aralkyle, un reste cycloalkyle ou un reste hétérocyclique, ces restes pouvant être substitués par des groupes fonctionnels.

3. Utilisation d'acides bisphosphoniques et de leurs dérivés pour la fabrication de médicaments pour la prophylaxie et/ou le traitement de maladies autoimmunes et/ou d'allergies, **caractérisée en ce que** sont utilisés comme acides bisphosphoniques et leurs dérivés :
- des acides bisphosphoniques et leurs dérivés de formule générale 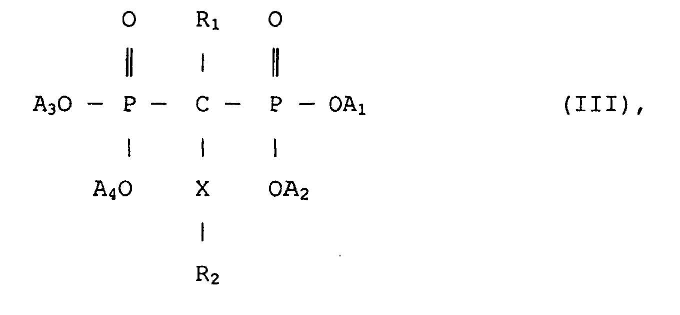 dans laquelle peuvent signifier :
A₁, A₂, A₃, A₄, qui peuvent être identiques ou différents : un hydrogène, un reste alkyle ramifié ou non ramifié avec 1-12 atomes de carbone, un reste aryle, un reste aralkyle, un reste cycloalkyle ou un reste hétérocyclique, ces restes pouvant être substitués par des groupes fonctionnels, un métal du 1^{er}, 2^{ième} ou 3^{ième} groupe principal du système périodique, tels que Na, K, Ca Mg, Al, de même qu'ammonium ou composé d'ammonium substitué ou non substitué, qui sont dérivés de l'éthylène diamine ou d'acides aminés,
R₁ : H, OH,
X, qui peut également être supprimé : (CH₂)₁₋₅, amidino,
R₂: -NH₂ 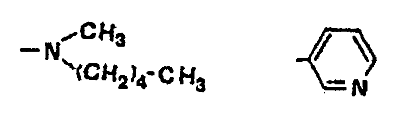 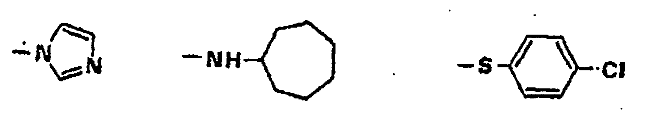
où l'utilisation des acides bisphosphoniques et/ou de leurs dérivés et/ou des composés aboutissant aux produits métaboliques ou de dégradation adéquats a lieu
en combinaison avec les auto-antigènes spécifiques pour la prophylaxie et le traitement de la maladie auto-immune correspondante ou
en combinaison avec les allergènes spécifiques pour la prophylaxie et le traitement de l'allergie correspondante
dans laquelle des fragments, dérivés ou analogues des auto-antigènes ou des allergènes ou encore des fragments d'analogues peuvent aussi être utilisés à la place des auto-antigènes ou des allergènes respectifs, dès lors que lesdites substances présentent respectivement les mêmes propriétés immunologiques que les molécules correspondantes des auto-antigènes ou allergènes prises dans leur ensemble.

4. Utilisation d'acides bisphosphoniques et de leurs dérivés en combinaison avec des auto-antigènes ou des allergènes pour la fabrication de médicaments pour la prophylaxie et/ou le traitement de maladies autoimmunes et/ou d'allergies selon l'une des revendications 1 à 3, **caractérisée en ce que** sont respectivement utilisés pour le traitement de :
sclérose en plaques protéine basique de la myéline
(sclérose multiple) (PBM), autres extraits du tissu du système nerveux ;
arthrite rhumatoïde collagène de type I, II, III ;
Thyroïdite chronique de Hashimoto thyroglobuline ;
Myasthénie d'Erbprotéine du récepteur
Goldflam (Myasthénia gravis) d'acétylcholine ;
lupus érythémateux ADN ;
diabète sucré extraits de cellules des îlots pancréatiques, insuline humaine ;
cirrhose biliaire primaire extraits hépatiques ;
hépatite chronique extraits de cellules
active hépatiques ;
Adrénalite (Adrénalitis)/maladie d'Addison extraits de corticosurrénale ;
Polymyosite extraits cutanés, extraits musculaires ;
Dermatomyosite extraits musculaires et/ou cutanés ;
anémie autohémolytique extraits de cellules de lignée hématopoïétique ;
inflammation cardiaque ou du myocarde extraits cardiaques ;
sclérodermie extraits cutanés, extraits de cellules cutanées ;
uvéite (uvéite phaco antigénique protéine du cristallin ; antigène S ;
"Phakouveitis", ophtalmie sympathique) mélange d'antigène S ;
pemphigus vulgaire extraits cutanés ;
pemphigoïde extraits cutanés ;
anémie pernicieuse extraits de cellules gastriques,
extraits de cellules pariétales,
facteur intrinsèque ;
gastrite atrophique extraits de cellules
auto-immune gastriques ;
maladie de Crohn extraits intestinaux ;
allergies liées à une extraits intestinaux,
colite ulcéreuse allergènes spécifiques à l'allergie.

5. Utilisation d'acides bisphosphoniques et de leurs dérivés pour la fabrication de médicaments pour la prophylaxie et/ou le traitement de maladies autoimmunes et/ou d'allergies selon l'une des revendications 1 à 3, **caractérisée en ce que** les préparations des combinaisons sont appliquées sous forme solide, en tant que pommade, solution ou spray.
